# EUROPEAN PATENT APPLICATION

(11) **EP 4 810 068 A1**
(43) Date of publication of application: **23.09.2026**
(21) Application number: 24890820.4
(22) Date of filing: 15.11.2024
(51) Int. Cl.: A61K 48/00, A61K 47/12, A61K 35/761, A61K 47/26, A61P 27/02, A61K 47/18

(54) **PHARMACEUTICAL COMPOSITION OF RECOMBINANT ADENO-ASSOCIATED VIRUS AND USE THEREOF**

(30) Priority: 15.11.2023 CN 202311518844
(71) Applicant: Chengdu Origen Biotechnology Co., Ltd., Chengdu, Sichuan 610037 (CN)
(72) Inventor: KE, Xiao, Chengdu, Sichuan 610037 (CN); ZHENG, Qiang, Chengdu, Sichuan 610037 (CN); JIANG, Hao, Chengdu, Sichuan 610037 (CN); LUO, Junbo, Chengdu, Sichuan 610037 (CN)
(74) Representative: reuteler & cie SA
(86) International application number: PCT/CN2024/132332
(87) International publication number: WO 2025/103468

(57) **Abstract**

Provided is a pharmaceutical composition, which pharmaceutical composition comprising a recombinant adeno-associated virus (AAV) and a buffer, a stabilizer and a nonionic surfactant. The pharmaceutical composition has good stability, thereby ensuring the stability of the product quality.

## Description

### FIELD

The present disclosure relates to the technical field of biology, in particular to a pharmaceutical composition of recombinant adeno-associated virus and use thereof.

### BACKGROUND

Adeno-associated virus (AAV) belongs to the Parvoviridae family and the Dependovirus genus. The recombinant adeno-associated virus (rAAV) carrying therapeutic genes has been widely used in gene transduction, gene therapy, vaccination, oncolytic therapy and other fields, it has many advantages, such as low pathogenicity, wide infection tissue, wide range of host cells (can be infected and expressed in proliferating and non-proliferating cells), low immunogenicity, long-term expression of exogenous genes in vivo, and no host cell genome integration, thus it is widely used in experimental and clinical research.

During the development process of rAAV products, in addition to consideration of safety and effectiveness of the virus itself, it is also necessary to consider stability of the product preparation, such as the time and possible environmental changes (temperature, stress, illumination, etc.) during the manufacturing, transportation, storage and administration process. Therefore, in order to ensure the stability of product quality, there are requirements imposed on the preparation development and improvement.

### SUMMARY

The present disclosure aims to provide a stable pharmaceutical composition comprising a recombinant adeno-associated virus (AAV).

Specifically, the first aspect of the present disclosure provides a pharmaceutical composition, wherein the pharmaceutical composition comprises a recombinant adeno-associated virus (AAV) and a buffer, an amino acid, a stabilizer, and a nonionic surfactant.

In some embodiments, the buffer of the present disclosure is a citrate buffer solution. In some preferred embodiments, the citrate is a sodium citrate, such as a monovalent, divalent or trivalent salt of sodium citrate, preferably a trivalent salt of sodium citrate.

In some embodiments, the amino acid of the present disclosure is selected from aspartic acid, arginine, glycine, histidine, and proline; preferably, the amino acid is arginine or aspartic acid; more preferably, the amino acid is arginine.

In some embodiments, the stabilizer of the present disclosure is selected from sucrose, trehalose, mannitol, lactose, galactose, glucose, and maltose. In some preferred embodiments, the stabilizer of the present disclosure is selected from sucrose, trehalose, and mannitol. In some more preferred embodiments, the stabilizer of the present disclosure is selected from sucrose or trehalose.

In some embodiments, the non-ionic surfactant of the present disclosure is selected from poloxamer 188, polysorbate 20, polysorbate, polyethylene glycol-hydroxystearate (HS15), vitamin E polyethylene glycol succinate (TPGS). In some preferred embodiments, the non-ionic surfactant of the present disclosure is selected from poloxamer 188 (P188), polysorbate 20 (Tween 20), and polysorbate 80 (Tween 80).

In some embodiments, in the pharmaceutical composition of the present disclosure the concentration of the buffer solution is from 10mM to 200mM; the concentration of the amino acid is from 10mM to 200mM; the concentration of the stabilizer is from 1% to 20wt%; the concentration of the non-ionic surfactant is from 0.001% to 0.1wt%.

In some embodiments, in the pharmaceutical composition of the present disclosure, the concentration of the buffer solution is from 10mM to 100mM; the concentration of the amino acid is from 20mM to 150mM; the concentration of the stabilizer is from 2.5% to 10wt%; the concentration of the non-ionic surfactant is from 0.001% to 0.05wt%.

In some preferred embodiments, in the pharmaceutical composition of the present disclosure, the concentration of the buffer solution is from 20mM to 40mM; the concentration of the amino acid is from 40mM to 100mM; the concentration of the stabilizer is from 3% to 10wt%; the concentration of the non-ionic surfactant is from 0.001% to 0.05wt%.

In some embodiments, the pharmaceutical composition of the present disclosure comprises 40mM of a buffer solution; 40mM of an amino acid; 5%wt of a stabilizer; 0.001% to 0.05wt% of a non-ionic surfactant.

In some embodiments, the pharmaceutical composition of the present disclosure comprises 40mM of a buffer solution; 100mM of an amino acid; 5%wt of a stabilizer; 0.001% to 0.05wt% of a non-ionic surfactant.

In some embodiments, the pharmaceutical composition of the present disclosure comprises 40mM of a buffer solution; 60mM of an amino acid; 5%wt of a stabilizer; 0.001% to 0.05wt% of a non-ionic surfactant.

In some embodiments, the pharmaceutical composition of the present disclosure comprises 20mM of a buffer solution; 100mM of an amino acid; 5%wt of a stabilizer; 0.001% to 0.05wt% of a non-ionic surfactant.

In some embodiments, the pH of the pharmaceutical composition of the present disclosure is from 5.5 to 8.0. In some preferred embodiments, the pH of the pharmaceutical composition of the present disclosure is from 6.0 to 8.0. In some preferred embodiments, the pH of the pharmaceutical composition of the present disclosure is from 5.5 to 7.5. In some preferred embodiments, the pH of the pharmaceutical composition of the present disclosure is from 5.5 to 6.5. In some more preferred embodiments, the pH of the pharmaceutical composition of the present disclosure is 6.0±0.2. The pH of the pharmaceutical composition can be adjusted to the desired end-point pH by using sodium hydroxide or hydrochloric acid as the pH regulator.

In some embodiments, the capsid protein serotype of the recombinant adeno-associated virus of the present disclosure is selected from AAV1, AAV2, AAV3, AAV4, AAV5, AAV6, AAV7, AAV8, AAV9, AAV10, AAV11 or variants thereof. In some preferred embodiments, the capsid protein serotype of the recombinant adeno-associated virus of the present disclosure is an AAV8 capsid protein or a modified AAV8 capsid protein.

In some embodiments, the recombinant adeno-associated virus of the present disclosure comprises a coding sequence for encoding an anti-VEGF protein, wherein the anti-VEGF protein is a fusion protein comprising the amino acid sequence as set forth in SEQ ID NO: 1, or comprises a protein having at least 80%, 90%, 95% or 99% homology with the above-mentioned antibody or protein sequence.

In some embodiments, the recombinant adeno-associated virus of the present disclosure comprises:
(i) a rAAV capsid protein, wherein the capsid protein is an AAV8 capsid protein or a modified AAV8 capsid protein; and
(ii) a polynucleotide expression cassette, arranged in the 5' to 3' direction comprising:
   (a)an AAV 5'ITR;
   (b)a CBA promoter;
   (c)a Chi intron;
   (d)a Kozak sequence;
   (e) a nucleic acid encoding an anti-VEGF protein, wherein the nucleic acid has a nucleotide sequence as set forth in SEQ ID NO: 2;
   (f) a rabbit-globin polyadenylation signal;
   (g) an AAV 3'ITR.

In some specific embodiments, the polynucleotide expression cassette of the present disclosure comprises the nucleotide sequence set forth in SEQ ID NO: 3 from 5' to 3'.

In some specific embodiments, the capsid protein of the present disclosure is a modified AAV8 capsid protein, wherein the modified AAV8 capsid protein has a substitution with the polypeptide sequence as set forth in SEQ ID NO: 4 at amino acid positions 588 to 592 of the parent AAV8.

In some embodiments, the genomic concentration of the recombinant adeno-associated virus (AAV) in the pharmaceutical composition of the present disclosure is about 1*10⁷ vg/ml to 1*10¹⁴ vg/ml. In some preferred embodiments, the genomic concentration of a recombinant adeno-associated virus (AAV) of the present disclosure is about 1*10⁹vg/ml to 1*10¹³vg/ml; preferably 1*10⁹ vg/ml to 1*10¹² vg/ml; preferably 1*10¹⁰ vg/ml to 1*10¹³ vg/ml.

In some preferred specific embodiments, the genomic concentration of the recombinant adeno-associated virus (AAV) in the present disclosure is about 1 x 10⁷, 1.5 x 10⁷, 2 x 10⁷, 2.5 x 10⁷, 3 x 10⁷, 3.5 x 10⁷, 4 x 10⁷, 4.5 x 10⁷, 5 x 10⁷, 5.5 x 10⁷, 6 x 10⁷, 6.5 x 10⁷, 7 x 10⁷, 7.5 x 10⁷, 8 x 10⁷, 8.5 x 10⁷, 9 x 10⁷, 9.5 x 10⁷, 1 x 10⁸, 1.5 x 10⁸, 2 x 10⁸, 2.5 x 10⁸, 3 x 10⁸, 3.5 x 10⁸, 4 x 10⁸, 4.5 x 10⁸, 5 x 10⁸, 5.5 x 10⁸, 6 x 10⁸, 6.5 x 10⁸, 7 x 10⁸, 7.5 x 10⁸, 8 x 10⁸, 8.5 x 10⁸, 9 x 10⁸, 9.5 x 10⁸, 1 x 10⁹, 1.5 x 10⁹, 2 x 10⁹, 2.5 x 10⁹, 3 x 10⁹, 3.5 x 10⁹, 4 x 10⁹, 4.5 x 10⁹, 5 x 10⁹, 5.5 x 10⁹, 6 x 10⁹, 6.5 x 10⁹, 7 x 10⁹, 7.5 x 10⁹, 8 x 10⁹, 8.5 x 10⁹, 9 x 10⁹, 9.5 x 10⁹, 1 x 10¹⁰, 1.5 x 10¹⁰, 2 x 10¹⁰, 2.5 x 10¹⁰, 3 x 10¹⁰, 3.5 x 10¹⁰, 4 x 10¹⁰, 4.5 x 10¹⁰, 5 x 10¹⁰, 5.5 x 10¹⁰, 6 x 10¹⁰, 6.5 x 10¹⁰, 7 x 10¹⁰, 7.5 x 10¹⁰, 8 x 10¹⁰, 8.5 x 10¹⁰, 9 x 10¹⁰, 9.5 x 10¹⁰, 1 x 10¹¹, 1.5 x 10¹¹, 2 x 10¹¹, 2.5 x 10¹¹, 3 x 10¹¹, 3.5 x 10¹¹, 4 x 10¹¹, 4.5 x 10¹¹, 5 x 10¹¹, 5.5 x 10¹¹, 6 x 10¹¹, 6.5 x 10¹¹, 7 x 10¹¹, 7.5 x 10¹¹, 8 x 10¹¹, 8.5 x 10¹¹, 9 x 10¹¹, 9.5 x 10¹¹, 1 x 10¹², 1.5 x 10¹², 2 x 10¹², 2.5 x 10¹², 3 x 10¹², 3.5 x 10¹², 4 x 10¹², 4.5 x 10¹², 5 x 10¹², 5.5 x 10¹², 6 x 10¹², 6.5 x 10¹², 7 x 10¹², 7.5 x 10¹², 8 x 10¹², 8.5 x 10¹², 9 x 10¹², 9.5 x 10¹², 1 x 10¹³, 1.5 x 10¹³, 2 x 10¹³, 2.5 x 10¹³, 3 x 10¹³, 3.5 x 10¹³, 4 x 10¹³, 4.5 x 10¹³, 5 x 10¹³, 5.5 x 10¹³, 6 x 10¹³, 6.5 x 10¹³, 7 x 10¹³, 7.5 x 10⁸, 8x 10¹³, 8.5 x 10¹³, 9 x 10¹³, 9.5 x 10¹³, 1 x 10¹⁴ (all units are vg/ml).

In some embodiments, the pharmaceutical composition of the present disclosure is a preparation for intravitreal, subretinal, intrachoroidal, intravenous, intratumoral, or intramuscular injection. In some preferred embodiments, the pharmaceutical composition is a preparation for intravitreal, subretinal, or intrachoroidal injection.

In some embodiments, the pharmaceutical composition of the present disclosure is a liquid composition or a pharmaceutical composition obtained by lyophilizing a liquid composition.

In some embodiments, the pharmaceutical composition of the present disclosure is stored in a unit dose container. In some specific embodiments, the unit dose container is a vial or a syringe. In some preferred embodiments, the vial is a glass vial; in other preferred embodiments, the syringe is a pre-filled syringe.

Another aspect of the present disclosure provides a use of the pharmaceutical composition according to any one of the preceding claims in the preparation of a medicament for treating a disease associated with VEGF.

In some embodiments, the disease associated with VEGF is an ocular neovascular disease.

In some preferred embodiments, the ocular disease according to the present disclosure is selected from the group consisting of age-related macular degeneration, retinal neovascularization, choroidal neovascularization, diabetic retinopathy, proliferative diabetic retinopathy, retinal vein occlusion, central retinal vein occlusion, branch retinal vein occlusion, diabetic macular oedema, diabetic retinal ischemia, ischemic retinopathy and diabetic retinal oedema, macular oedema secondary to retinal vein occlusion, polypoidal choroidal vasculopathy, wet age-related macular degeneration with very low vision, choroidal neovascularization secondary to pathological myopia, neovascular glaucoma, iris neovascular disease, and retinopathy of prematurity.

Another aspect of the present disclosure provides a method for treating a disease, wherein the method comprises administering a therapeutically effective amount of the pharmaceutical composition with recombinant adeno-associated virus of the present disclosure to a patient/subject in need.

In some embodiments, the pharmaceutical composition of the present disclosure is administered by subcutaneous injection, intramuscular injection, intravenous injection, intratumoral injection, intravitreal injection, suprachoroidal injection or subretinal injection. In some preferred embodiments, the pharmaceutical composition of the present disclosure is administered by suprachoroidal injection or subretinal injection.

In some embodiments, the disease associated with VEGF is an ocular disease.

In some preferred embodiments, the ocular disease according to the present disclosure is selected from the group consisting of age-related macular degeneration, retinal neovascularization, choroidal neovascularization, diabetic retinopathy, proliferative diabetic retinopathy, retinal vein occlusion, central retinal vein occlusion, branch retinal vein occlusion, diabetic macular oedema, diabetic retinal ischemia, ischemic retinopathy and diabetic retinal oedema, macular oedema secondary to retinal vein occlusion, polypoidal choroidal vasculopathy, wet age-related macular degeneration with very low vision, choroidal neovascularization secondary to pathological myopia, neovascular glaucoma, iris neovascular disease, and retinopathy of prematurity.

### DEFINITIONS

In order to facilitate understanding of the present disclosure, certain technical and scientific terms are specifically defined below. Unless expressly defined otherwise herein, all other technical and scientific terms used herein have meanings commonly understood by one of average skill in the art.

The term 'Pharmaceutical composition' refers to a composition comprising one or more of the recombinant adeno-associated viruses (rAAV) described herein, together with other components such as a physiological/pharmaceutic vector and excipient. The purpose of the pharmaceutical composition is to facilitate the administration of drug to an organism, to enhance the absorption and/or expression of the active ingredient to achieve the desired biological activity. In this disclosure, "pharmaceutical composition" and "formulation" are not mutually exclusive.

As used herein, the term "AAV" refers to both naturally occurring adeno-associated virus and recombinant form of adeno-associated viruses (rAAV) and includes mutant form of AAV. The term AAV further includes but is not limited to AAV type 1, AAV type 2, AAV type 3, AAV type 4, AAV type 5, AAV type 6, AAV type 7, AAV type 8, AAV type 9, AAV type 10, avian AAV, bovine AAV, canine AAV, equine AAV, sheep AAV, primate AAV, and non-primate AAV. In some embodiments, AAV is AAV8.

Pharmaceutically acceptable buffer is well known in the art and includes, but is not limited to, inorganic acid such as phosphate (sodium or potassium), bicarbonate, etc; organic acid such as citrate, acetate, succinate, etc; acidic buffer such as acetic acid, phosphoric acid, hydrochloric acid, carbonic acid, succinic acid, citric acid, histidine hydrochloric acid, malic acid, etc; alkaline buffer, such as sodium hydroxide, Tris, HEPES, etc.

"Citrate" buffer is a buffer that includes citrate ions. Examples of citrate buffer include sodium citrate, potassium citrate, calcium citrate, magnesium citrate, etc. The preferred citrate buffer is sodium citrate.

Amino acids are well-known in the art and includes natural and unnatural amino acid (synthetic amino acid). Examples such as: alanine (Ala); valine (Val); leucine (Leu); isoleucine (Ile); proline (Pro); phenylalanine (Phe); tryptophan (Trp); methionine (Met); glycine (Gly); serine (Ser); threonine (Thr); cysteine (Cys); tyrosine (Tyr); asparagine (Asn); glutamine (Gln); selenocysteine (Sec); pyrrolysine (Pyl); lysine (Lys); arginine (Arg); histidine (His); aspartic acid (Asp); glutamic acid (Glu), etc.

It has been found that adding more than one sugar and/or sugar alcohol as a stabilizer at proper level (for example, between about 1% and about 10%) contributes to the stability of liquid preparation and/or freeze-dried preparation. Any sugar may be used as a stabilizer for use in the pharmaceutical composition of the present disclosure, non-limiting examples include: monosaccharides, disaccharides or polysaccharides, or water-soluble glucans, including, for example, fructose, glucose, mannose, sorbose, xylose, maltose, lactose, sucrose, glucan, trehalose, amylopectin, dextrin, cyclodextrin, soluble starch, hydroxyethyl starch, carboxymethyl cellulose and the like. Sugar alcohol is defined as hydrocarbon with about 4 to about 8 carbon atoms and one hydroxyl group. Non-limiting examples of sugar alcohol that may be used in the pharmaceutical composition provided by the present disclosure include: mannitol, sorbitol, inositol, galactitol, dulcitol, xylitol, and arabitol.

The primary nonionic surfactants suitable for use in the pharmaceutical compositions ofthe invention is known in the pharmaceutical field, which includes, but not limited to polysorbate 80 (Tween 80; PS80), polysorbate 20(Tween 20; PS20) and various poloxamer or pluronics, including pluronic F-68 and BRIJ 35, or mixture thereof.

"Lyophilized preparation" means a preparation or pharmaceutical composition obtained following a vacuum freeze-drying step undergone by a pharmaceutical composition in liquid or solution form or a preparationf liquid or solution.

In representative embodiments, the pharmaceutical composition of the present disclosure has a physiologically compatible pH value. In representative aspects, pH of the pharmaceutical composition is from about 5.0 to about 8.0, from about 5.5 to about 8.0, from about 6.0 to about 8.0, from about 5.5 to about 7.5, from about 6.0 to about 7.5, from about 6.5 to about 7.5. In certain embodiments, the pH of the preparation is about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0. In representative aspects, pH of the pharmaceutical composition is about 6.0 or about 6.5. In some embodiments, pH of the pharmaceutical composition is about 6.0±0.5. In some embodiments, pH of the pharmaceutical composition is about 6.0±0.2.

As used herein, the term "about" refers to an approximate range of positive or negative 10% from a specified value. For example, the expression "about 20%" includes a range of 18-22%. As used herein, "about" also includes the exact value. Therefore, "about 20%" means "about 20%" and "20%".

The preparation or pharmaceutical composition of the present disclosure comprises additional pharmaceutically acceptable ingredients. In representative aspects, the preparation or pharmaceutical composition comprises any one or a combination of the following: an acidifier, an anticoagulant, an antibiosis preservative, an antioxidant, a preservative, a base, an inorganic salt, etc.

An "effective amount" includes an amount sufficient to ameliorate or prevent a symptom or disorder of a medical condition. An effective amount also means an amount sufficient to permit or facilitate a diagnosis. The effective amount used for a particular subject or veterinary subject may vary depending on factors such as the condition to be treated, the general health of the subject, the method and dose of administration, and the severity of side effects. The effective amount may be the maximum dose or dosing regimen that avoids significant side effects or toxic effects.

The terms "patient" and "subject" are used interchangeable, and in their normal sense, to refer to an organism, including human and non-human animals, suffering from or susceptible to a condition that can be prevented or treated by administering the compositions of the present disclosure. Examples of subjects include, but are not limited to: human, chimpanzees, other ape and monkey species; farm animal such as cattle, sheep, pig, goat, and horse; domestic mammals such as dog and cat; laboratory animals, including rodent such as mouse, rat, and guinea pig; birds including poultry, wild bird, and game bird, such as chicken, turkey and other quail, ducks, goose, etc. The term does not indicate a particular age. Thus, adults, adolescents, and newborn individuals are all subjects.

"Stable" or "substantially stable" may refer to the pharmaceutical composition maintaining its properties (e.g., pH, Osmotic pressure, genome titer, capsid protein purity, rAAV purity, activity, insoluble particles, relative protein expression, and the like maintain stable) over a long storage period. For example, a composition that is stable for storage does not have significant impurities due to degradation of the composition over a long period of time and maintains a high purity. For example, it may have impurities less than 10%, or less than 9%, or less than 8%, or less than 7%, or less than 6%, or less than 5%, or less than 4%, or less than 3%, or less than 2%, or less than 1% of degradation products over a long period of time. In some cases, a composition that is stable for storage has little or a very limited amount of insoluble particles of ≥10 µm, or ≥25 µm, or ≥ 50 µm, or ≥100 µm, or ≥150 µm, or ≥200 µm, or ≥250 µm, or ≥300 µm, or ≥350 µm, or ≥400 µm, or ≥450 µm, or ≥500 µm, or even larger over a long period of time. In some cases, a composition that is stable in storage substantially maintain its activity over extended periods of time, for example, the composition maintains 100%, or more than 99%, or more than 98%, or more than 97%, or more than 96%, or more than 95%, or more than 94%, or more than 93%, or more than 92%, or more than 91%, or more than 90%, or more than 85%, or more than 80%, or more than 75% of its activity. A long time period of is a period of time such as more than 1 week, or more than 2 weeks, or more than 3 weeks, or more than 1 month, or more than 2 months, or more than 3 months, or more than 4 months, or more than 6 months, or more than 9 months, or more than 1 year, or more than 1.5 years (e.g., 18 months), or more than 2 years, or more than 2.5 years (e.g., 30 months), or more than 3 years or more, or more than 3.5 years (e.g., 42 months), more than 4 years, or more than 4.5 years (e.g., 54 months), or more than 5 years. In some embodiments, the stored stable pharmaceutical composition is substantially stable over an extended period of time at ambient temperature, such as a temperature of 20 to 40 °C, or 25 to 35 °C, or 25 to 30 °C. In some cases, the storage-stable composition is substantially stable for a longer period of time at a temperature below ambient temperature, for example, the temperature below ambient temperature is 0 to 20 °C, or 0 to 15 °C, or 0 to 10 °C, or 2 to 8 °C.

In some cases, the term "stable" or "substantially stable" can also refer to the thermal stability of the AAV in the pharmaceutical composition resistant to high temperatures or temperature changes. Two pathways are involved in the thermal denaturation of AAV: genome ejection and capsid disruption. Genome ejection of the AAV capsid occurs at relatively low temperatures and DNA begins to escape from the intact capsid. AAV capsid disruption occurs when protein unfolding causes the viral capsid to lose structural integrity and break. To monitor or evaluate the thermal stability of AAV, conventional experimental methods in this filed such as Elisa, qPCR, AAV-ID, and high-throughput thermal stability analysis can be used. In some embodiments, the present disclosure utilizes the Uncle (Unchained Labs) multifunctional protein stability analysis system to monitor the genome ejection and capsid disruption of AAV to evaluate the thermal stability of the protein. Tracking genome ejection by using DNA binding fluorescent dyes such as SYBR Gold (nucleic acid dyes are able to combine with nucleic acids ejected from the AAV and emit fluorescence, the more nucleic acids ejected, the stronger the fluorescence signal after binding), and determining the melting temperature (Tm) based on DNA release. A higher Tm temperature indicates better thermal stability of the AAV. In some cases, Tm of AAV in the pharmaceutical composition of the present disclosure is increased by at least 0.5 °C, or at least 1 °C, or at least 1.5 °C, or at least 2 °C, or at least 2.5 °C, or at least 3 °C, or at least 3.5 °C, or at least 4 °C, or at least 4.5 °C, or at least 5 °C or more relative to other compositions. The disruption of AAV capsid can be studied on Uncle by monitoring the intrinsic protein fluorescence of capsid proteins without dyes (when the protein is heated to a certain point, the protein unravels, the hydrophobic region is exposed, and the protein autofluorescence changes, so the protein structure can be determined by changes in the protein autofluorescence). The capsid protein of the AAV in the pharmaceutical composition in the invention undergoes little structural change.

### BRIEF DESCRIPTION OF THE DRAWINGS

FIG. 1 shows isothermal stability test results of the samples in Example 2.
FIG. 2 shows isothermal stability test results of the samples of Example 2 after 5 freeze-thaw cycles.
FIG. 3 shows isothermal stability test results of the samples in Example 3.
FIG. 4 shows isothermal stability test results of the samples of Example 3 after 5 freeze-thaw cycles.
FIG. 5 shows isothermal stability test results of the samples in Example 5.

### DETAILED DESCRIPTION

The present disclosure will be further described below with reference to specific examples. It should be understood that these examples only serve to illustrate the present disclosure, instead of imposing limitations to the scope of the present disclosure.

### Example 1 Preparation of recombinant adeno-associated virus vector (rAAV) stock solution

A recombinant plasmid containing the target protein gene described in the present disclosure was constructed and cloned in *Escherichia coli* using conventional DNA recombination techniques and cloning techniques and the plasmid was subsequently used for the preparation of recombinant AAV virus.

Firstly, a polynucleotide expression cassette and a recombinant plasmid encoding the target gene were constructed. Exemplarily, in 5' to 3' order, the expression cassette or plasmid included: an AAV 5'ITR, a CBA promoter, a Chi intron, a Kozak sequence, a nucleic acid of the coding sequence encoding anti-VEGF protein, a rabbit-globin polyadenylation signal, and an AAV 3'ITR. The coding sequence for encoding the anti-VEGF protein in the polynucleotide expression cassette and the recombinant plasmid were used to encode the aflibercept fusion protein, wherein aflibercept has the amino acid sequence as set forth in SEQ ID NO: 1.

The recombinant adeno-associated virus vector (rAAV) was prepared by a three-plasmid co-transfection process generally known in the art. The AAV capsid used in the example was a modified AAV8 capsid protein, which has a substitution with the polypeptide sequence as set forth in SEQ ID NO: 4 (RGNQQNTARQ) at amino acid positions 588 to 592 (QQNTA) of the parent AAV8. The construction of the plasmid containing the capsid protein was specifically described in detail in the related patent PCT/CN2022/142185 (Title of Invention: MODIFIED AAV CAPSID PROTEIN AND USE THEREOF), which was specifically and entirely incorporated herein by reference.

AAV virus packaging was generated by co-transfecting cells with three plasmids including: a first plasmid comprising the ITR flanked by the anti-VEGF protein coding sequence constructed as described above; a second plasmid encoding the Rep/Cap gene as described above; and a third plasmid containing adenovirus helper genes. After transfection, cells were collected, lysed to release the virus, and the virus titer was measured by PCR for use.

### Example 2

The recombinant adeno-associated virus prepared in Example 1 was used to produce preparation samples according to the following Table 1, wherein the concentration of rAAV was 1×10¹² vg/mL.

**Table 1**

| Sucrose | Preparation 1 | Preparation 2 | Preparation 3 | Preparation 4 | Preparation 5 | Preparation 6 | Preparation 7 | Preparation 8 | Preparation 9 | Preparation 10 |
|---|---|---|---|---|---|---|---|---|---|---|
| Trehalose | 5% | 5% | 5% | 5% | 5% | 5% | 5% | 5% | 5% | 5% |
| Aspartic acid | / | 40 mM | / | | 40 mM | / | | 40 mM | / | |
| Arginine | / | / | 40 mM | / | / | 40 mM | / | / | 40 mM | 100 mM |
| Proline | 40 mM | / | / | 40 mM | / | / | 40 mM | / | / | / |
| Sodium citrate | / | / | / | / | / | / | 40 mM | 40 mM | 40 mM | 40 mM |
| HEPES (Hydroxyethyl piperazine ethane sulfonic acid) | / | / | / | 40 mM | 40 mM | 40 mM | / | / | / | / |
| Na₂HPO₄ 2H₂O | 8 mM | 8 mM | 8 mM | / | / | / | / | / | / | / |
| KH₂PO₄ | 2 mM | 2 mM | 2 mM | / | / | / | / | / | / | / |
| NaCl | 60 mM | 60 mM | 60 mM | 100 mM | 100 mM | 100 mM | 40 mM | 40 mM | 40 mM | / |
| KCl | 3 mM | 3 mM | 3 mM | / | / | / | / | / | / | / |
| Tween 20 | 0.05% | 0.05% | 0.05% | 0.05% | 0.05% | 0.05% | 0.05% | 0.05% | 0.05% | 0.05% |
| Osmotic pressure (mOsm/L) | / | / | 345 | / | / | 440 | 387 | 401 | 422 | 456 |
| pH value | 7 | 7 | 7 | 7 | 7 | 7 | 6 | 6 | 6 | 6 |

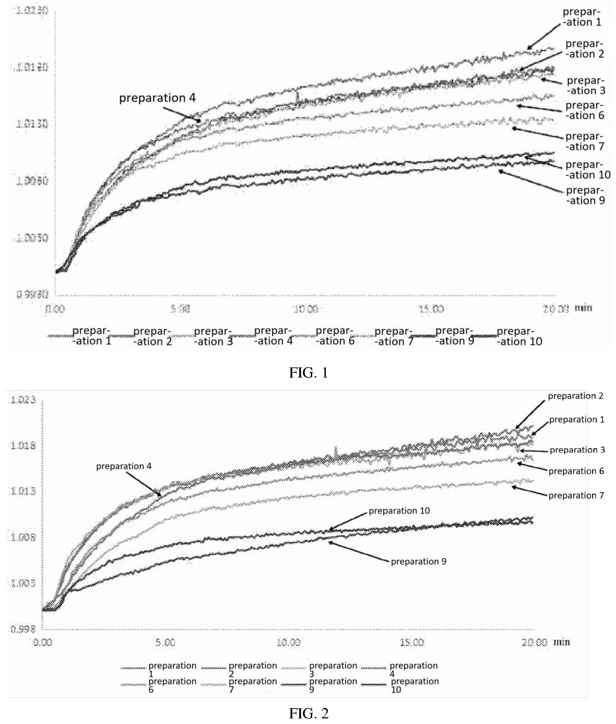

The thermal stability of rAAV in the above-mentioned preparation solution was verified by isothermal stability assays. In the isothermal stability assay, the sample was kept at a specific temperature for a period of time (e.g., 18h, 24h), and the capsid protein structure changes (e.g., aggregation/denaturation) of the sample rAAV were detected in real time by means of fluorescence, static light scattering (SLS), dynamic light scattering (DLS) and the like. The specific operations were as follows: 9µl of the above-mentioned preparation sample was added into the Uni tube, and the assays were performed by selecting the program "Isothermal" from "Isothermal Toolbox" in the high-throughput multi-parameter protein temperature analysis system (UNcle, UNCHAINED LABS). The parameters of the high-throughput multi-parameter protein temperature analysis system were as follows: temperature 30 °C, constant temperature time: 5min, investigation time: 20h, UV 266 Filter 1 (0.50), Blue Laser Filter 3 (0.25). The thermal stability change condition of protein samples in different preparations over time (abscissa) were observed by the relative BCM (300-430 nm) response value (ordinate). The results were shown in FIG. 1. By superimposing the time-varying curves of the samples to be compared, and observing the early and late, and the rapid and slow rise of the curves, the early and late and the rapid and slow condition of the aggregation/denaturation of the samples can be known, thus the stability of the samples can be evaluated. As illustrated by the thermal stability curves in FIG. 1, the stability profiles/curves changes of the citric acid buffer system preparations (preparations 6-10) exhibited a more gradual change compared to those of the phosphate buffer system preparations (preparations 1-3) and the HPEPS buffer system preparations (preparations 4-6), and their thermal stability was relatively better. Furthermore, in the citric acid buffer system preparations, the addition of arginine (preparations 9-10) was more helpful in improving the stability of the preparations than proline (preparation 7).

At the same time, the preparation samples were subjected to 5 freeze-thaw cycles, and the isothermal thermal stability of the samples was examined again by the high-throughput multi-parameter protein temperature analysis system, and the conditions were the same as those of the non-frozen-thawed samples. The results were shown in FIG. 2. It can also be seen from FIG. 2 that, consistent with before freezing and thawing, after experiencing freeze-thaw cycles, the curve changes of the citric acid buffer system preparations (preparations 6-10) exhibited a more gradual change compared to those of the phosphate buffer system preparations (preparations 1-3) and the HPEPS buffer system preparations (preparations 4-6), and their stability was relatively better, among which the preparations 9-10 exhibited the best stability.

### Example 3

The recombinant adeno-associated virus prepared in Example 1 was used to produce preparation samples according to the following Table 2, wherein the concentration of rAAV was 1×10¹² vg/mL.

**Table 2**

| Sucrose | Preparation 11 | Preparation 12 | Preparation 13 | Preparation 14 | Preparation 15 |
|---|---|---|---|---|---|
| Sodium citrate | 40mM | 40mM | 40mM | 40mM | 20mM |
| Arginine | 60 mM | / | / | 60 mM | 100 mM |
| Proline | / | 40 mM | / | / | / |
| Aspartic acid | / | / | 40 mM | / | / |
| Sucrose | / | / | / | 5% | 5% |
| Trehalose | 5% | 5% | 5% | / | / |
| Tween 20 | 0.05% | 0.05% | 0.05% | 0.05% | 0.05% |
| pH value | 6 | 6 | 6 | 6 | 6 |
| Osmotic pressure (mOsm/L) | 389 | 324 | 336 | 400 | 414 |

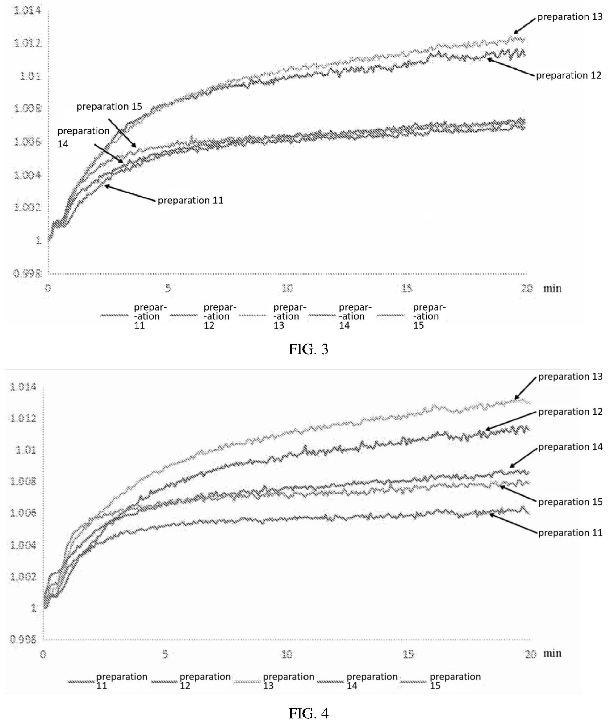

The thermal stability of rAAV in the above-mentioned preparation solution was verified by the isothermal stability assays. In the isothermal stability assay, the sample was kept at a specific temperature for a period of time (e.g., 18h, 24h), and the capsid protein structure changes (e.g., aggregation/denaturation) of the sample rAAV were detected in real time by fluorescence, static light scattering (SLS), dynamic light scattering (DLS) and the like. The specific operations were as follows: 9µl of the above-mentioned preparation sample was added into the Uni tube, and the assays were performed by selecting the program "Isothermal" from "Isothermal Toolbox" in the high-throughput multi-parameter protein temperature analysis system (UNcle, UNCHAINED LABS). The parameters set for the high-throughput multi-parameter protein temperature analysis system were as follows: temperature: 30 °C, constant temperature time: 5min, investigation time: 18h, UV 266 Filter 1 (0.50), Blue Laser Filter 3 (0.25). The thermal stability change condition of protein samples in different preparations over time (abscissa) were observed by the relative BCM (300-430 nm) response value (ordinate). The results were shown in FIG. 3. By superimposing the time-varying curves of the samples to be compared, and observing the early and late, and the rapid and slow rise of the curves, the early and late and the rapid and slow condition of the aggregation/denaturation of the samples can be known, thus the stability of the samples can be evaluated. As illustrated by FIG. 3, in the citric acid buffer system preparations, the curve changes of the preparations with added arginine (preparations 11, 14-15) exhibited a more gradual change compared to the preparations with added proline and aspartic acid (preparations 12-13), and their thermal stability was relatively better.

At the same time, the preparation samples were subjected to 5 freeze-thaw cycles, and the isothermal thermal stability of the preparation samples was examined again by the high-throughput multi-parameter protein temperature analysis system, and the conditions were the same as those of the non-frozen-thawed preparation samples. The results were shown in FIG. 4. Consistent with before freezing and thawing, after experiencing freeze-thaw cycles, the curve changes of the preparations with added arginine (preparations 11, 14-15) exhibited a more gradual change compared to those of the preparations with added proline and aspartic acid (preparations 12-13), and their thermal stability was relatively better.

### Example 4

The recombinant adeno-associated virus prepared in Example 1 was used to produce preparation samples according to the following Table 3, wherein the concentration of rAAV was 1×10¹² vg/mL.

**Table 3**

| Types of auxiliary materials | Preparation 16 | Preparation 17 | Preparation 18 |
|---|---|---|---|
| Arginine | 40mM | 40mM | 40mM |
| Sodium citrate | 40mM | 40mM | 40mM |
| P188 | 0.002% | 0.002% | 0.002% |
| pH value | 6 | 7 | 8 |

Evaluation of Stability: The Tm value of the capsid protein in each group of preparations was determined using the Uncle (Unchained Labs) system, and the Tm value of the gene ejection after adding nucleic acid dyes. At the same time, each group of samples was frozen and thawed for 5 times (the sample was placed in a low temperature ≤ -65 °C for ≥ 2 hours, and placed in room temperature for ≥ 0.5 hours, the operations formed 1 cycle), nucleic acid dyes were then added to test the Tm value after freezing and thawing.

Test method for Tm value of capsid protein in the preparation: 9 µl of the above-mentioned preparation sample was taken and added into the Uni tube, the "Tm, Tagg & Optional DLS" program in the high-throughput multi-parameter protein temperature analysis system (UNcle, UNCHAINED LABS) was then selected for carrying out the real-time detection. The parameters set for the high-throughput multi-parameter protein temperature analysis system were as follows: an initial temperature of 15 °C, an incubation time of 180s, a temperature-rise rate: 0.5 °C/min, final temperature of 95 °C, UV 266 Opaque (0.00), Blue Laser open (1.00).

The specific operations for testing the Tm value of gene ejection after adding nucleic acid dye were as follows: firstly, the rAAV samples were treated by adding nucleic acid dye, and then the gene ejection from the sample in a certain temperature range (25 °C-95 °C) was real-time detected by fluorescence, static light scattering (SLS), dynamic light scattering (DLS), etc. The specific operations were as follows: 20X SYBR^{™} GLOD nucleic acid dye was added into the above-mentioned preparation sample, and 9µl was taken and added into the Uni tube, then real-time detection was performed by selecting the program "Tm, Tagg & Optional DLS" in the high-throughput multi-parameter protein temperature analysis system (UNcle, UNCHAINED LABS). The parameters of the high-throughput multi-parameter protein temperature analysis system were set as follows: starting temperature 15 °C, incubation time 180s, heating rate: 0.5 °C/min, termination temperature 95 °C, UV 266 Opaque (0.00), Blue Laser open (1.00).

The results were shown in Table 4. As can be seen, the preparation at a pH of 6 had a better stability than the rAAV preparation at a pH of 7 and a pH of 8.

**Table 4**

| | Preparation 16 | Preparation 17 | Preparation 18 |
|---|---|---|---|
| Capsid protein Tm(°C) | 62.95 | 58.49 | 58.7 |
| Gene ejection Tm(°C) | 60.47 | 57.08 | 57.57 |

### Example 5

The recombinant adeno-associated virus prepared in Example 1 was used to produce preparation samples according to the following Table 5, wherein the concentration of rAAV was 1 ×10¹³ vg/mL.

**Table 5**

| Formulation composition | Preparation 15 | Preparation 19 | Preparation 20 | Preparation 21 |
|---|---|---|---|---|
| Na₂HPO₄ ·H₂O | | 8mM | / | / |
| KH₂PO₄ | | 2mM | / | / |
| NaCl | | 60mM | 100mM | / |
| KCl | | 3mM | / | / |
| HEPES | | / | 40mM | / |
| Arginine | 100mM | 40mM | 40mM | / |
| Sodium citrate | 20mM | / | / | 15mM |
| Glycine | | / | / | 15mM |
| Mannitol | | / | / | 2% |
| Trehalose | | 5% | 5% | 1% |
| Sucrose | 5% | | | |
| Tween 20 | 0.05% | 0.05% | 0.05% | / |
| Tween 80 | | / | / | 0.005% |
| pH | 6 | 6 | 6 | 6 |

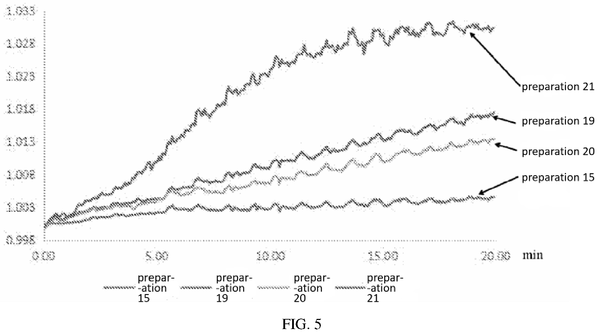

The thermal stability of rAAV in the above-mentioned preparation solution was verified by isothermal stability assays. In the isothermal stability assay, the sample was kept at a specific temperature for a period of time (e.g., 18h, 24h), and the capsid protein structure changes (e.g., aggregation/denaturation) of the sample rAAV were detected in real time by means of fluorescence, static light scattering (SLS), dynamic light scattering (DLS) and the like. The specific operations were as follows: 9µl of the above-mentioned preparation sample was added into the Uni tube, and the assays were performed by selecting the program "Isothermal" from "Isothermal Toolbox" in the high-throughput multi-parameter protein temperature analysis system (UNcle, UNCHAINED LABS). The parameters of the high-throughput multi-parameter protein temperature analysis system were as follows: temperature 30 °C, constant temperature time 5min, investigation time 18h, UV 266 Filter 1 (0.50), Blue Laser Filter 3 (0.25). The thermal stability change condition of protein samples in different preparations over time (abscissa) were observed by the relative BCM (300-430 nm) response value (ordinate). The results were shown in FIG. 5. By superimposing the time-varying curves of the samples to be compared, and observing the early and late, and the rapid and slow rise of the curves, the early and late and the rapid and slow condition of the aggregation/denaturation of the samples can be known, thus the stability of the samples can be evaluated. As illustrated by the thermal stability curves in FIG. 5, the curve changes of the preparation15 exhibited a more gradual change compared to those of the preparations preparations 19-21, and their thermal stability was relatively better.

After the preparations formulas in Table 5 were placed at 25 °C for 5 days, their protein expression quantity was tested. The method included: HEK293 cell suspension (with a density of 1×10⁵ cells/ml) was added into a 96-well plate in an amount of 100µl/well, and cultured overnight in a 37 °C, 5% carbon dioxide incubator. Subsequently, the 96-well plate was taken out, the preparation sample solution diluted to a concentration of 1×10¹⁰ vg/ml was added in an amount of 100µl/well, and placed in a 37 °C, 5% carbon dioxide incubator for 24 hours. The 96-well plate was then taken out, the supernatant in the well was discarded, 100µl of DMEM complete culture medium and 100µl of Luciferease colorimetric solution restored to room temperature were sequentially added to each well, and placed at room temperature and a light-proof condition for 5 minutes. Finally, the sample was subjected to the Luciferease colorimetric reading using a microplate reader. Each group of samples was placed in a refrigerator ≤ -65 °C and subjected to the synchronous testing, and the results were used as the sample data of the control group. The ratio of the test results of the sample after being placed at 25 °C for 5 days to the control group sample was the relative protein expression. The results were shown in Table 6.

**Table 6**

| | Preparation 15 | Preparation 19 | Preparation 20 | Preparation 21 |
|---|---|---|---|---|
| Relative protein expression (%) | 100.1 | 43.3 | 60.8 | 61.8 |

### Example 6

The recombinant adeno-associated virus prepared in Example 1 was used to produce preparation samples according to the following Table 7, wherein the concentration of rAAV was 1×10¹³ vg/mL.

**Table 7**

| Sucrose | Preparation 22 | Preparation 23 | Preparation 24 | Preparation 15 |
|---|---|---|---|---|
| Sodium citrate | 20mM | 20mM | 20mM | 20mM |
| Arginine | / | / | / | 100 mM |
| Histidine | / | / | 100 mM | |
| Proline | / | 100 mM | / | / |
| Aspartic acid | 100mM | / | / | / |
| Sucrose | 5% | 5% | 5% | 5% |
| Tween 20 | 0.05% | 0.05% | 0.05% | 0.05% |
| pH value | 6 | 6 | 6 | 6 |

After the preparations in Table 7 were placed at 25 °C for 5 days, their protein expression quantities were tested. The method included: HEK293 cell suspension (with a density of 1×10⁵ cells/ml) was added into a 96-well plate in an amount of 100µl/well, and cultured overnight in a 37 °C, 5% carbon dioxide incubator. Subsequently, the 96-well plate was taken out, the preparation sample solution diluted to a concentration of 1×10¹⁰ vg/ml was added in an amount of 100µl/well, and placed in a 37 °C, 5% carbon dioxide incubator for 24 hours. The 96-well plate was then taken out, the supernatant in the well was discarded, 100µl of DMEM complete culture medium and 100µl of Luciferease colorimetric solution restored to room temperature were sequentially added to each well, and placed at room temperature and a light-proof condition for 5 minutes. Finally, the samples were subjected to the Luciferease colorimetric reading using a microplate reader. Each group of samples was placed in a refrigerator ≤ -65 °C and subjected to the synchronous testing, and the results were used as the sample data of the control group. The ratio of the test results of the sample after being placed at 25 °C for 5 days to the control group sample was exactly the relative protein expression. The results were shown in Table 8.

**Table 8**

| | Preparation 22 | Preparation 23 | Preparation 24 | Preparation 15 |
|---|---|---|---|---|
| Relative protein expression (%) | 91.8 | 81.6 | 67.4 | 100 |

### Example 7

The long-term stability (at a temperature ≤ -65 °C for 12 months and freezing-thawing for 3 months) of the prepared preparation sample 15 comprising the anti-VEGF protein target gene with a concentration specification of 1.0×10¹³ vg/mL was investigated. The investigation indicators included genome titer, relative protein expression after infecting cells, and biological activity of protein after infecting cells, as shown in Table 9.

**Table 9**

| Test name | Test | Sampling | Detection | | |
|---|---|---|---|---|---|
| | condition | time point | indicators | | |
| | | | Genome titer (vg/mL) | Relative protein expression after infection of cells | Protein biological activity after infection of cells |
| Long term | ≤-65°C | 0 | 2.73E+12 | 100% | 104% |
| | | 3 | 2.87E+12 | 97% | 99% |
| | | 6 | 2.54E+12 | 99% | 97% |
| | | 9 | 2.63E+12 | 96% | 121% |
| | | 12 | 2.93E+12 | 97% | 101% |
| Freezing and thawing | ≤-65°C~ 25°C±2°C | 0 | 2.87E+12 | 97% | 99% |
| | | 1 | 2.34E+12 | 113% | 97% |
| | | 2 | 2.32E+12 | 109% | 100% |
| | | 3 | 2.32E+12 | 107% | 96% |

## Claims

1. A pharmaceutical composition comprising a recombinant adeno-associated virus, **characterized in that** the pharmaceutical composition further comprises a buffer solution, an amino acid, a stabilizer, and a non-ionic surfactant, wherein the buffer solution is a citrate buffer solution, preferably, the citrate is a sodium citrate; the amino acid is selected from aspartic acid, arginine, glycine, histidine, and proline, preferably, the amino acid is arginine; the stabilizer is selected from sucrose, trehalose, mannitol, lactose, galactose, glucose, and maltose, preferably sucrose or trehalose; the non-ionic surfactant is selected from poloxamer 188, polysorbate 20, polysorbate 80, HS15, and TPGS, preferably poloxamer 188, polysorbate 20, and polysorbate 80.

2. The pharmaceutical composition according to any one of the preceding claims, **characterized in that** in the pharmaceutical composition, the concentration of the buffer is from 10mM to 200mM, the concentration of the amino acid is from 10mM to 200mM, the concentration of the stabilizer is from 1% to 20wt%, and the concentration of the non-ionic surfactant is from 0.001% to 0.1wt%; preferably, in the pharmaceutical composition, the concentration of the buffer solution is from 10mM to 100mM, the concentration of the amino acid is from 20mM to 150mM, the concentration of the stabilizer is from 2.5% to 10wt%, and the concentration of the non-ionic surfactant is from 0.001% to 0.05wt%; more preferably, in the pharmaceutical composition, the concentration of the buffer solution is from 20mM to 40mM, the concentration of the amino acid is from 40mM to 100mM, the concentration of the stabilizer is from 3% to 10wt%, and the concentration of the non-ionic surfactant is from 0.001% to 0.05wt%.

3. The pharmaceutical composition according to any one of the preceding claims, **characterized in that** the pharmaceutical composition comprises 40mM of a buffer, 40mM of an amino acid, 5wt% of a stabilizer, and 0.001% to 0.05wt% of a non-ionic surfactant; or the pharmaceutical composition comprises 40mM of a buffer solution, 100mM of an amino acid, 5wt% of a stabilizer, and 0.001% to 0.05wt% of a non-ionic surfactant; or the pharmaceutical composition comprises 40mM of a buffer solution, 60mM of an amino acid, 5wt% of a stabilizer, and 0.001% to 0.05wt% of a non-ionic surfactant; or the pharmaceutical composition comprises 20mM of a buffer solution, 100mM of an amino acid, 5wt% of a stabilizer, and 0.001% to 0.05wt% of a non-ionic surfactant.

4. The pharmaceutical composition according to any one of the preceding claims, **characterized in that** the pH of the pharmaceutical composition is from 5.5 to 8.0; preferably, the pH of the pharmaceutical composition is from 6.0 to 8.0; preferably, the pH is from 5.5 to 6.5; preferably, the pH is 6.0±0.2.

5. The pharmaceutical composition according to any one of the preceding claims, **characterized in that** the capsid protein serotype of the recombinant adeno-associated virus is an AAV8 capsid protein or a modified AAV8 capsid protein.

6. The pharmaceutical composition according to any one of the preceding claims, **characterized in that** the recombinant adeno-associated virus comprises a coding sequence for encoding an anti-VEGF protein, and the anti-VEGF protein is a fusion protein comprising the amino acid sequence as set forth in SEQ ID NO: 1; preferably, the recombinant adeno-associated virus comprises:
(i) a rAAV capsid protein, wherein the capsid protein is an AAV8 capsid protein or a modified AAV8 capsid protein; and
(ii) a polynucleotide expression cassette, arranged in the 5' to 3' direction comprising:
(a) an AAV 5'ITR;
(b) a CBA promoter;
(c) a Chi intron;
(d) a Kozak sequence;
(e) a nucleic acid encoding an anti-VEGF protein, wherein the nucleic acid has a nucleotide sequence as set forth in SEQ ID NO: 2;
(f) a rabbit-globin polyadenylation signal;
(g) an AAV 3'ITR;
More preferably, the polynucleotide expression cassette comprises the nucleotide sequence set forth in SEQ ID NO: 3 from 5' to 3'.

7. The pharmaceutical composition according to claim 5, **characterized in that** the capsid protein is a modified AAV8 capsid protein, wherein the modified AAV8 capsid protein has a substitution with the polypeptide sequence as set forth in SEQ ID NO: 4 at amino acid positions 588 to 592 of the parent AAV8.

8. The pharmaceutical composition according to any one of the preceding claims, **characterized in that** the pharmaceutical composition is an intravitreal injection preparation, a subretinal injection preparation, an intrachoroidal injection preparation, an intravenous injection preparation, an intratumoral injection preparation or an intramuscular injection preparation; preferably, the pharmaceutical composition is an intravitreal injection preparation, a subretinal injection preparation, or an intrachoroidal injection preparation.

9. The pharmaceutical composition according to any one of the preceding claims, **characterized in that** the pharmaceutical composition is a liquid preparation.

10. The pharmaceutical composition according to any one of the preceding claims, **characterized in that** the pharmaceutical composition is a lyophilized preparation.

11. The pharmaceutical composition according to any one of the preceding claims, **characterized in that** the pharmaceutical composition is stored in a unit dose container.

12. The pharmaceutical composition according to claim 11, **characterized in that** the unit dose container is a vial or a syringe.

13. The pharmaceutical composition according to claim 12, **characterized in that** the vial is a glass vial.

14. The pharmaceutical composition according to claim 12, **characterized in that** the syringe is a pre-filled syringe.

15. The pharmaceutical composition according to any one of the preceding claims, **characterized in that** genome concentration of the recombinant adeno-associated virus in the pharmaceutical composition is about 1*10⁷ vg/ml to 1*10¹⁴ vg/ml; preferably, genome concentration of the recombinant adeno-associated virus is about 1*10⁹ vg/ml to 1*10¹³ vg/ml; more preferably, genome concentration of the recombinant adeno-associated virus is about 1*10⁹ vg/ml to 1*10¹² vg/ml.

16. Use of the pharmaceutical composition according to any one of the preceding claims in the preparation of a medicament for treating a disease associated with VEGF.

17. A method for treating a disease associated with VEGF, comprising administering a therapeutically effective amount of the pharmaceutical composition according to any one of claims 1-15 to a patient/subject in need.

18. The use according to claim 16 or the method according to claim 17, **characterized in that** the disease associated with VEGF is an ocular neovascular disease.

19. The use according to claim 16 or the method according to claim 17, **characterized in that** the ocular neovascular disease is selected from the group consisting of age-related macular degeneration, retinal neovascularization, choroidal neovascularization, diabetic retinopathy, proliferative diabetic retinopathy, retinal vein occlusion, central retinal vein occlusion, branch retinal vein occlusion, diabetic macular oedema, diabetic retinal ischemia, ischemic retinopathy and diabetic retinal oedema, macular oedema secondary to retinal vein occlusion, polypoidal choroidal vasculopathy, wet age-related macular degeneration with very low vision, choroidal neovascularization secondary to pathological myopia, neovascular glaucoma, iris neovascular disease, and retinopathy of prematurity.
